# EUROPÄISCHE PATENTSCHRIFT

(11) **EP 0 711 575 B1**
(45) Veröffentlichungstag und Bekanntmachung des Hinweises auf die Patenterteilung: **28.08.2002**
(21) Anmeldenummer: 95850196.7
(22) Anmeldetag: 08.11.1995
(51) Int. Cl.: A61N 1/05, A61B 5/0408, A61B 5/0416, A61B 5/0478, A61B 5/0492

(54) **Verfahren zur Kontaktierung von Glaskohlenstoff-Elektroden**
Method of electrical contacting of vitreous carbon electrodes
Procédé pour établir un contact électrique pour des électrodes en carbone vitreux

(30) Priorität: 09.11.1994 DE 4440001
(43) Veröffentlichungstag der Anmeldung: 15.05.1996
(73) Patentinhaber: St. Jude Medical AB, 175 84 Järfälla (SE)
(72) Erfinder: Tegeder, Volker, D-91054 Erlangen (DE)

(56) Entgegenhaltungen:
- EP-A- 0 173 860
- DE-A- 2 613 072
- DE-A- 3 337 470
- US-A- 1 181 811

## Beschreibung

Die Erfindung betrifft ein Verfahren zur Kontaktierung von Glaskohlenstoff-Elektroden.

Elektroden aus Glaskohlenstoff eignen sich als implantierbare Aktoren und Sensoren in der Biomedizin (siehe dazu DE-PS 26 13 072); sie besitzen nämlich eine gute Bioverträglichkeit und können eine hohe Kapazität aufweisen, wobei die Materialkosten - im Vergleich zu anderen Elektrodenmaterialien - gering sind. Unter Aktoren bzw. Effektoren werden Elektroden verstanden, die eine Reizwirkung ausüben, beispielsweise Reizelektroden für Herzschrittmacher; Sensoren sind Elektroden, mit denen gemessen wird, beispielsweise Elektroden zur Bestimmung von Glucose.

Glaskohlenstoff-Elektroden werden im allgemeinen in der Weise hergestellt, daß aus einem Harz-Vorkondensat durch Härten ein aus vernetztem Kunstharz bestehender Elektrodenrohling erzeugt und dieser durch Pyrolyse in die eigentliche Elektrode übergeführt wird. Bei der Pyrolyse, die - unter inerter Atmosphäre - im allgemeinen bei Temperaturen zwischen 700 und 2000°C erfolgt, wird aus dem vernetzten Kunstharz Glaskohlenstoff gebildet (siehe dazu beispielsweise EP-A-0 140 172). Elektrodenrohlinge können durch mechanische Bearbeitung relativ leicht in die gewünschte Form gebracht werden, die elektrische Kontaktierung von Glaskohlenstoff-Elektroden ist aber schwierig.

Die Kontaktierung von implantierbaren Glaskohlenstoff-Elektroden, die einen Elektrodenkopf und einen Elektrodenschaft aufweisen, erfolgt im allgemeinen in der Weise, daß auf den Elektrodenschaft eine Platinhülse aufgesteckt oder aufgepreßt wird. Eine derartige Kontaktierung ist aber nicht nur aufwendig in der Herstellung, sondern auch mechanisch wenig belastbar. Bedingt durch konstruktive Merkmale von Aktoren bzw. Sensoren, beispielsweise eine schlauchförmige Isolation, ist nämlich der Durchmesser des Elektrodenschaftes begrenzt. So beträgt beispielsweise, jeweils bei einem Elektrodendurchmesser von 2,37 mm, der Durchmesser des Elektrodenschaftes bei einer Herzschrittmacherelektrode 1,34 mm und bei der Meßelektrode eines katheterförmigen Glucosesensors 1 mm. Aufgrund der mechanischen Eigenschaften von Glaskohlenstoff, d.h. der Sprödigkeit, ist deshalb - bei einer Kontaktierung dieser Art - die Belastbarkeit gegen Zug und Scherung sehr gering. So beträgt beispielsweise die Zugbelastbarkeit der Glaskohlenstoff-Elektrode eines Glucosesensors der vorstehend genannten Art lediglich 2,75 N (280 p), dann bricht der Elektrodenschaft. Somit wird eine derartige Kontaktierung bei der Handhabung der Elektroden, beispielsweise beim Zusammenbau des Sensors, durch einen Scherbruch des Glaskohlenstoffs leicht zerstört. Außerdem ist dabei eine an sich wünschenswerte Verkleinerung des Sensors nicht zu realisieren, weil die Kontaktierung dann noch empfindlicher wäre.

Aufgabe der Erfindung ist es, ein Verfahren anzugeben, das eine einfache und mechanisch stabile elektrische Kontaktierung von Glaskohlenstoff-Elektroden, insbesondere bei katheterförmigen Aktoren und Sensoren, erlaubt.

Dies wird erfindungsgemäß durch ein Verfahren erreicht, bei dem ein Elektrodenrohling aus einem vernetzten Kunstharz mit einer den Elektrodenkörper teilweise durchdringenden Bohrung versehen wird, in die Bohrung ein massiver oder hohler Stift aus einem biokompatiblen, hochschmelzenden Metall eingeführt wird, dessen Außendurchmesser kleiner ist als der Durchmesser der Bohrung und der aus dem Elektrodenkörper herausragt, und der Elektrodenrohling einer Pyrolyse unterworfen wird, wobei das vernetzte Kunstharz in Glaskohlenstoff übergeführt wird und der Elektrodenkörper auf den Stift aufschrumpft.

Alternativ dazu kann zur Lösung der genannten Aufgabe auch in der Weise vorgegangen werden, daß ein Elektrodenrohling aus einem vernetzten Kunstharz mit einer den Elektrodenkörper vollständig durchdringenden Bohrung versehen wird, daß in die Bohrung eine Hülse aus einem biokompatiblen, hochschmelzenden Metall eingeführt wird, deren Außendurchmesser kleiner ist als der Durchmesser der Bohrung und die auf beiden Seiten aus dem Elektrodenkörper herausragt, und daß der Elektrodenrohling einer Pyrolyse unterworfen wird, wobei das vernetzte Kunstharz in Glaskohlenstoff übergeführt wird und der Elektrodenkörper auf die Hülse aufschrumpft.

Bei der Pyrolyse des vernetzten Kunstharzes, bei der aus dem Kunstharz Glaskohlenstoff entsteht, erfolgt eine Schrumpfung, die bis zu etwa 25 % betragen kann. Die Abmessungen des Stiftes bzw. der Hülse werden deshalb so gewählt, daß der Elektrodenkörper bei der Pyrolyse darauf aufschrumpft, oder anders ausgedrückt: das Innenmaß der Bohrung (ohne Stift bzw. Hülse) nach der Pyrolyse, d.h. dem Schrumpfungsvorgang, ist kleiner als das Außenmaß des Stiftes bzw. der Hülse. Auf diese Weise ergibt sich ein ausgezeichneter Verbund zwischen Elektrodenkörper und Stift bzw. Hülse.

Es hat sich als vorteilhaft erwiesen, wenn der Durchmesser des Stiftes in dem in der Bohrung befindlichen Bereich bzw. der Außendurchmesser der Hülse etwa 5 bis 15 % kleiner ist als der Durchmesser der Bohrung. Vorzugsweise sind die Abmessungen von Stift bzw. Hülse etwa 10 % kleiner als das Innenmaß der Bohrung.

Bei einer Kontaktierung gemäß der Erfindung, bei der die "Schwachstelle" Elektrodenschaft entfällt, wird eine Biegebelastung vom Metall aufgenommen; sie wird dann entweder elastisch aufgefangen oder sie führt zu einer Verformung ohne Bruch. Was die mechanische Zugbelastung anbetrifft, so können - bei einem Außendurchmesser der Elektroden von 2,3 mm - Belastungen von mehr als 19,6 N (2 kp) erreicht werden. Vorteilhaft ist ferner, daß - zusätzlich zur sehr viel höheren Belastbarkeit des Kontaktes - der Verfahrensschritt der Kontaktierung nach der Pyrolyse entfällt, wodurch sich einerseits eine vereinfachte Herstellung ergibt und andererseits die Elektrodenoberfläche geschützt wird, weil sie nach der Pyrolyse - und gegebenenfalls nachfolgender Aktivierung - nicht mehr berührt werden muß. Mit dem Verfahren nach der Erfindung können außerdem wesentlich kleinere Elektroden kontaktiert werden als dies bislang der Fall war.

Bei der Kontaktierung mittels eines Stiftes ist es vorteilhaft, wenn sich der Stift in dem in der Bohrung befindlichen Bereich konisch nach innen erweitert, d.h. in den Elektrodenkörper hinein. Außerdem ist von Vorteil, wenn sich auch die Bohrung selbst konisch nach innen erweitert. Auf diese Weise kann nämlich die Belastbarkeit noch weiter gesteigert werden. Ferner weist der Stift in dem aus dem Elektrodenkörper herausragenden Bereich vorteilhaft einen größeren Querschnitt auf als in dem in der Bohrung befindlichen Bereich. Dadurch wird nämlich das Aufbringen einer schlauchförmigen Isolierung erleichtert.

Bei der Kontaktierung nach der Erfindung besteht der Stift bzw. die Hülse vorteilhaft aus einem Metall mit einem Schmelzpunkt ≥ 1500°C. Vorzugsweise finden folgende Metalle (Schmelzpunkt) Verwendung: Titan (1727°C), Platin (1772°C), Tantal (3030°C) und Wolfram (3380°C). Stift bzw. Hülse können beispielsweise aber auch aus einer Platin/Iridium-Legierung bestehen.

Die Pyrolyse des vernetzten Kunstharzes erfolgt vorzugsweise bei einer Temperatur ≥ 1000°C, und zwar in einer inerten Atmosphäre; als Schutzgas dient beispielsweise Stickstoff oder Argon. Als Ausgangsmaterialien zur Herstellung des Glaskohlenstoffs dienen übliche Harz-Vorkondensate, insbesondere Harze aus vorkondensiertem Furfurylalkohol; es können beispielsweise aber auch Vorkondensate von Phenolformaldehydund Furanharzen eingesetzt werden. Diese Harz-Vorkondensate werden dann vernetzt bzw. gehärtet.

Anhand eines Ausführungsbeispieles soll die Erfindung noch näher erläutert werden.

Ein an einem Ende abgerundeter zylindrischer Elektrodenkopf aus einem vernetzten Kunstharz hat beispielsweise einen Durchmesser von 2,94 mm sowie eine Höhe von 3,33 mm und weist eine zentrale Bohrung mit einem Durchmesser von 0,99 mm und einer Länge von 2,17 mm auf. Zur Kontaktierung dient ein zylinderförmiger Stift aus Platin, der im Bereich außerhalb der Bohrung eine Länge von 5,0 mm sowie einen Durchmesser von 1,5 mm aufweist und im Bereich innerhalb der Bohrung eine Länge von 1,65 mm sowie einen sich stetig von 0,86 mm auf 0,89 mm erweiternden Durchmesser. Der Durchmesser des Stiftes am Stiftende innerhalb der Bohrung (0,89 mm) ist somit ca. 10 % geringer als der Durchmesser der Bohrung (0,99 mm).

Wird der Elektrodenrohling, d.h. der vorstehend beschriebene Elektrodenkopf, einer Pyrolyse bei einer Temperatur von etwa 1100°C unterworfen, so schrumpft der Elektrodenkopf auf den Platinstift auf, wobei ein fester Verbund entsteht, d.h. eine dauerhafte und stabile Kontaktierung.

## Patentansprüche

1. Verfahren zur Kontaktierung von Glaskohlenstoff-Elektroden, **dadurch gekennzeichnet, daß** ein Elektrodenrohling aus einem vernetzten Kunstharz mit einer den Elektrodenkörper teilweise durchdringenden Bohrung versehen wird, daß in die Bohrung ein massiver oder hohler Stift aus einem biokompatiblen, hochschmelzenden Metall eingeführt wird, dessen Außendurchmesser kleiner ist als der Durchmesser der Bohrung und der aus dem Elektrodenkörper herausragt, und daß der Elektrodenrohling einer Pyrolyse unterworfen wird, wobei das vernetzte Kunstharz in Glaskohlenstoff übergeführt wird und der Elektrodenkörper auf den Stift aufschrumpft.

2. Verfahren nach Anspruch 1, **dadurch gekennzeichnet, daß** sich der Stift in dem in der Bohrung befindlichen Bereich konisch nach innen erweitert.

3. Verfahren nach Anspruch 2, **dadurch gekennzeichnet, daß** sich die Bohrung konisch nach innen erweitert.

4. Verfahren nach einem der Ansprüche 1 bis 3, **dadurch gekennzeichnet, daß** der Stift in dem aus dem Elektrodenkörper herausragenden Bereich einen größeren Querschnitt aufweist als in dem in der Bohrung befindlichen Bereich.

5. Verfahren zur Kontaktierung von Glaskohlenstoff-Elektroden, **dadurch gekennzeichnet, daß** ein Elektrodenrohling aus einem vernetzten Kunstharz mit einer den Elektrodenkörper vollständig durchdringenden Bohrung versehen wird, daß in die Bohrung eine Hülse aus einem biokompatiblen, hochschmelzenden Metall eingeführt wird, deren Außendurchmesser kleiner ist als der Durchmesser der Bohrung und die auf beiden Seiten aus dem Elektrodenkörper herausragt, und daß der Elektrodenrohling einer Pyrolyse unterworfen wird, wobei das vernetzte Kunstharz in Glaskohlenstoff übergeführt wird und der Elektrodenkörper auf die Hülse aufschrumpft.

6. Verfahren nach einem der Ansprüche 1 bis 5, **dadurch gekennzeichnet, daß** der Durchmesser des Stiftes in dem in der Bohrung befindlichen Bereich bzw. der Außendurchmesser der Hülse etwa 5 bis 15 % kleiner ist als der Durchmesser der Bohrung.

7. Verfahren nach einem der Ansprüche 1 bis 6, **dadurch gekennzeichnet, daß** das Metall einen Schmelzpunkt ≥ 1500°C besitzt.

8. Verfahren nach einem der Ansprüche 1 bis 7, **dadurch gekennzeichnet, daß** der Stift bzw. die Hülse aus Titan, Tantal, Wolfram, Platin oder einer Platin/Iridium-Legierung besteht.

9. Verfahren nach einem der Ansprüche 1 bis 8, **dadurch gekennzeichnet, daß** die Pyrolyse bei einer Temperatur ≥ 1000°C durchgeführt wird.

10. Verfahren nach einem der Ansprüche 1 bis 9, **dadurch gekennzeichnet, daß** ein Kunstharz aus einem vorkondensierten und vernetzten Furfurylalkohol verwendet wird.

## Claims

1. Method of contacting glass carbon electrodes, **characterised in that** an electrode blank made from a cross-linked synthetic resin is provided with a bore which passes partly through the electrode body, that there is introduced into the bore a solid or hollow pin which is made from a biocompatible high-melting metal, has a smaller external diameter than the diameter of the bore and projects out of the electrode body, and that the electrode blank is subjected to pyrolysis, whereby the cross-linked synthetic resin is converted into glass carbon and the electrode body shrinks onto the pin.

2. Method as claimed in Claim 1, **characterised in that** the pin widens conically inwards in the region located in the bore.

3. Method as claimed in Claim 2, **characterised in that** the bore widens conically inwards.

4. Method as claimed in one of Claims 1 to 3, **characterised in that** in the region which projects out of the electrode body the pin has a greater cross-section than in the region located in the bore.

5. Method of contacting glass carbon electrodes, **characterised in that** an electrode blank made from a cross-linked synthetic resin is provided with a bore which passes completely through the electrode body, that there is introduced into the bore a sleeve which is made from a biocompatible high-melting metal, has a smaller external diameter than the diameter of the bore and projects on both sides out of the electrode body, and that the electrode blank is subjected to pyrolysis, whereby the cross-linked synthetic resin is converted into glass carbon and the electrode body shrinks onto the sleeve.

6. Method as claimed in one of Claims 1 to 5, **characterised in that** the diameter of the pin in the region located in the bore or the external diameter of the sleeve is approximately 5 to 15 % smaller than the diameter of the bore.

7. Method as claimed in one of Claims 1 to 6, **characterised in that** the metal has a melting point = 1500 °C.

8. Method as claimed in one of Claims 1 to 7, **characterised in that** the pin or the sleeve is made from titanium, tantalum, tungsten, platinum or a platinum/iridium alloy.

9. Method as claimed in one of Claims 1 to 8, **characterised in that** the pyrolysis is carried out at a temperature = 1000 °C.

10. Method as claimed in one of Claims 1 to 9, **characterised in that** a synthetic resin made from a pre-condensed and cross-linked furfuryl alcohol is used.

## Revendications

1. Procédé d'établissement d'un contact électrique pour des électrodes en carbone vitreux, **caractérisé en ce qu'**il consiste à munir une ébauche d'électrode, en une résine de matière plastique réticulée, d'un trou traversant au moins en partie le corps de l'électrode, à introduire dans le trou une broche pleine ou creuse en un métal biocompatible et à point de fusion élevé, dont le diamètre extérieur est plus petit que le diamètre du trou et qui fait saillie du corps de l'électrode, et à soumettre l'ébauche d'électrode à une pyrolyse, la résine de matière plastique réticulée se transformant en du carbone vitreux et le corps de l'électrode se frettant sur la broche.

2. Procédé suivant la revendication 1, **caractérisé en ce que** la broche s'évase coniquement vers l'intérieur dans la partie se trouvant dans le trou.

3. Procédé suivant la revendication 2, **caractérisé en ce que** le trou s'évase coniquement vers l'intérieur.

4. Procédé suivant l'une des revendications 1 à 3, **caractérisé en ce que** la broche a, dans la partie faisant saillie du corps de l'électrode, une section transversale plus grande que dans la partie se trouvant dans le trou.

5. Procédé d'établissement d'un contact électrique pour des électrodes en carbone vitreux, **caractérisé en ce qu'**il consiste à munir une ébauche d'électrode en une résine de matière plastique réticulée, d'un trou traversant entièrement le corps de l'électrode, à introduire dans le trou une douille en un métal biocompatible et à point de fusion élevé, dont le diamètre extérieur est inférieur au diamètre du trou et qui fait saillie des deux côtés du corps de l'électrode et à soumettre l'ébauche d'électrode à une pyrolyse, la résine réticulée se transformant en carbone vitreux et le corps de l'électrode se frettant sur la douille.

6. Procédé suivant l'une des revendications 1 à 5, **caractérisé en ce que** le diamètre de la broche dans la partie se trouvant dans le trou, ou le diamètre extérieur de la douille, est plus petit d'environ 5 à 15 % que le diamètre du trou.

7. Procédé suivant l'une des revendications 1 à 6, **caractérisé en ce que** le métal a un point de fusion ≥ 1 500°C.

8. Procédé suivant l'une des revendications 1 à 7, **caractérisé en ce que** la broche ou la douille est en titane, en tantale, en tungstène, en platine ou en un alliage de platine et d'iridium.

9. Procédé suivant l'une des revendications 1 à 8, **caractérisé en que** qu'il consiste à effectuer la pyrolyse à une température ≥ 1 000°C.

10. Procédé suivant l'une des revendications 1 à 9, **caractérisé en ce qu'**il consiste à utiliser une résine de matière plastique en un alcool furfurylique précondensé et réticulé.
